# EUROPEAN PATENT APPLICATION

(11) **EP 1 454 631 A1**
(43) Date of publication of application: **08.09.2004**
(21) Application number: 04251253.3
(22) Date of filing: 04.03.2004
(51) Int. Cl.: A61K 38/28, A61K 47/36, A61K 9/00

(54) **Oral insulin composition and methods of making and using thereof**

(30) Priority: 04.03.2003 US 451245 P; 05.05.2003 US 467601 P; 09.05.2003 US 469017 P; 15.08.2003 US 495097 P
(71) Applicant: The Technology Development Company Ltd., Hamilton HM FX (BM)
(72) Inventor: Sabetsky, Vladimir, 105221 Saint-Petersburg (RU)
(74) Representative: Cockerton, Bruce Roger

(57) **Abstract**

A method of lowering blood glucose in a mammal includes orally administering a therapeutically effective amount of crystallized dextran microparticles and insulin to the mammal to lower blood glucose of the mammal. The composition may be a one phase or a structured multiphase composition for controlled release of insulin.

## Description

### FIELD OF THE INVENTION

This application claims benefit of the following U.S. Provisional Applications Serial Nos. 60/451,245, filed March 4, 2003; 60/467,601 filed May 5, 2003; 60/469,017 filed May 9, 2003; and 60/495,097 filed August 15, 2003, the disclosures of which are incorporated by reference herein in their entirety.

The present invention relates generally to insulin compositions and specifically to an oral insulin composition containing insulin and crystallized dextran microparticles.

### BACKGROUND OF THE INVENTION

Dextrans are high molecular weight polysaccharides synthesized by some micro organisms or by biochemical methods. Dextran with average molecular weight of about 75 kDa has a colloid osmotic pressure similar to blood plasma, so its aqueous solutions are used clinically as plasma expanders. Cross-linked dextrans in the form of beads are the basis for "Sephadex"® that is used in the GPC of proteins and for "Cytodex"® developed by Pharmacia to fulfill the special requirements of a micro-carrier cell culture. For example, U.S. Patent Nos. 6,395,302 and 6,303,148 (Hennink et al.) disclose attaching various biomaterials to cross-linked dextran particles. However, beads based on cross-linked dextran generally cannot be used for implant manufacturing owing to their potential toxicity due to the application of cross-linking agents (Blain J.F., Maghni K., Pelletier S. and Sirois P. Inflamm. Res. 48 (1999): 386-392).

U.S. Patent No. 4,713,249 (Schroder) describes a method of producing a depot matrix for biologically active substances. According to this patent, the depot matrix allegedly consists of carbohydrate microparticles, stabilized by crystallization, which implies using noncovalent bonds. The following process for producing the alleged crystallized carbohydrate microparticles is described by Schroder. A solution of a polymeric carbohydrate and a biologically-active substance is formed in one or more hydrophilic solvents. Then the mixture of the carbohydrate and the biologically active substance is emulsified in a liquid hydrophobic medium to form spherical droplets. The emulsion is then introduced into a crystallizing medium comprising acetone, ethanol or methanol to form spheres having a non-covalently cross-linked crystalline polymeric carbohydrate matrix, said matrix incorporating 0.001-50% by weight of the biologically-active substance. Thus, the biologically active substance is provided into the solution prior to crystallizing the spheres. Schroder does not describe the microstructure of the microparticles made by the multi-step method. Schroder's multi-step method is complex and uses organic solvents that are potentially toxic to cells and need to be removed. Furthermore, Schroder's composition is designed for injection, which is an inconvenient and painful method of administering the composition.

### BRIEF SUMMARY OF THE INVENTION

A method of lowering blood glucose in a mammal includes orally administering a therapeutically effective amount of crystallized dextran microparticles and insulin to the mammal to lower blood glucose of the mammal. The composition may be a one phase or a structured multi-phase composition for controlled release of insulin.

### BRIEF DESCRIPTION OF THE DRAWINGS

Figure 1 is a photograph of crystallized dextran microparticles spontaneously formed in 55.0% (W/W) aqueous solution of dextran with MW 70.0 kDa.

Figure 2A is a photograph of a cross-section of crystallized dextran microparticles shown in Figure 1.

Figure 2B is a photograph of a cross-section of a microparticle shown in Figure 2A. Microporous structure of the microparticle can be seen.

Figure 3 is a photograph of aggregates of crystallized dextran microparticles.

Figure 4 is a photograph of a slow release of the fluorescently labeled macromolecules from the implant which includes crystallized dextran microparticles into mouse muscle tissue on the 14^{th} day after intermuscular injection.

Figure 5 is a photograph of an emulsion of aqueous solution of PEG in aqueous solution of dextran (MW 500 kDa) containing crystallized dextran microparticles shown in Figure 1.

Figure 6 is a photograph of an emulsion of aqueous solution of dextran (MW 500 kDa) containing crystallized dextran microparticles shown in Figure 1 in aqueous solution of PEG.

Figure 7 is a photograph of an intramuscular injection of emulsion of aqueous solution of PEG in aqueous solution of dextran (MW 500 kDa) containing crystallized dextran microparticles shown in Figure 1.

Figure 8 is a photograph of a subcutaneous injection of emulsion of aqueous solution of PEG in aqueous solution of dextran (MW 500 kDa) containing crystallized dextran microparticles shown in Figure 1.

Figures 9A and 9C schematically illustrate partition behavior of different types of particles and phases in an aqueous two phase system.

Figure 9B is a photograph of a cross section of an implant structure based on the two phase system.

Figure 10 schematically illustrates therapeutic agent delivery methods according to an embodiment of the present invention.

### DETAILED DESCRIPTION OF THE PREFERRED EMBODIMENTS

The present inventor discovered that a composition of porous (i.e., microporous) microparticles, such as crystallized dextran microparticles, may be used as a vehicle for oral delivery of insulin. The composition may be a one phase composition or a multi-phase composition which forms a structured implant in a mammal.

The first section below describes the preferred crystallized dextran microparticles, the second section describes formation of the structured implant from a multi-phase composition, and the following sections describe specific examples of oral administration of the composition into mammals and methods of making the oral insulin composition.

### A. Crystallized Dextran Microparticles

The present inventor has experimentally found that crystallized dextran microparticles with an average diameter ranging from 0.5 to 3.5 microns were spontaneously formed in concentrated aqueous solutions of dextrans (40 - 65 % W/W) with molecular weights ranging from 1.0 to 200.0 kDa, at temperature ranging from 20 - 90 °C. If it is desired to form the microparticles at room temperature, then 2 to 18 kDa dextran solutions may be used. Of course, the microparticles may also be formed from 2 to 18 kDa solutions at temperatures above room temperature, if desired. The microparticles may be spontaneously formed from higher molecular weight dextran solutions, such as 20 to 75 kDa solutions, at higher temperatures above room temperature, such as about 40 to about 70°C. The microparticles may have any suitable shape such as a regular or an irregular shape, but are preferably spherical in shape, and are preferably 10 microns in diameter or less, such as 0.5 to 5 microns.

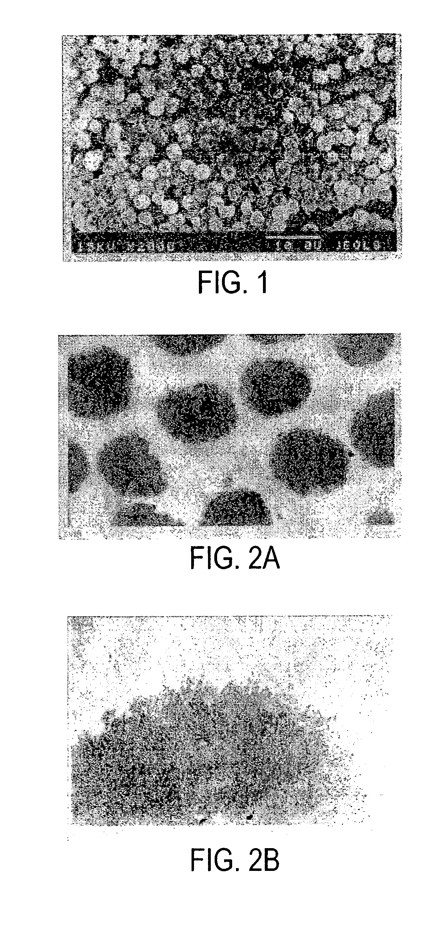

Transmission Electron Microscopy revealed the microporous structure of the crystallized dextran microparticles (see Figures 2A, 2B). Preferably, the microparticle porosity is at least 10 percent by volume, such as about 10 to about 50 percent, more preferably about 20 to about 40 percent. Thus, the structure comprises microporous microparticles with areas of macroporosity located between the particles.

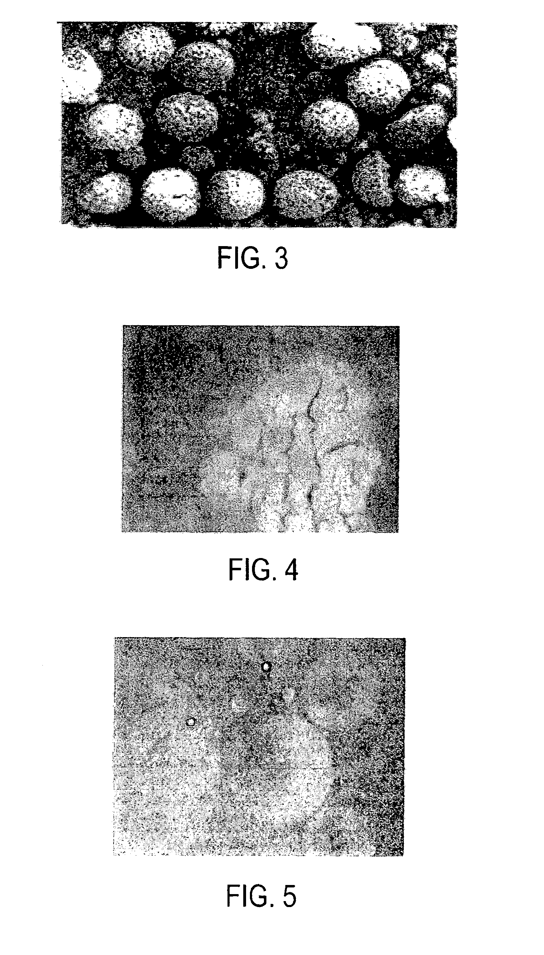

Spray drying of aqueous suspensions of the crystallized dextran microparticles has shown the possibility to produce substantially spherical aggregates of crystallized dextran microparticles with a diameter ranging from 10.0 to 150.0 microns (see Figure 3).

A non limiting example of a method of forming the dextran microparticles is as follows. 50.0 g of dextran T40 (40 kDa molecular weight) from Amersham Biosciences is added to 50.0 g of sterile distilled water in a 500 ml lab beaker to obtain 50% w/w solution under laminar flow. The mixture is stirred at 60°C (water bath) on a magnetic stirrer at 50 rpm until the dextran is completely dissolved and a clear solution is obtained. The solution can be vacuumed to remove all air inclusions. The clear solution is placed in lab oven at 60°C under a Tyvek"® lid. 3.5 hours later, a turbid viscous suspension is developed as a result of formation of crystallized dextran microparticles.

To eliminate non-crystallized dextran, the microparticles are washed by centrifugation, for example 3,000 g, 30 min, with 3 × 250 ml of distilled sterile water, or by filtration of diluted suspension of microparticles, for example one part microparticles and 10 parts water (3 × 250 ml of distilled sterile water through sterilization filter). The centrifugation/washing is done under laminar flow. The microparticles are placed in 500 ml lab beaker under a Tyvek® lid and dried at 60°C in lab oven for 8 hours to reach a moisture level of about 5%. The resulting dry powder consists of particles with a mean diameter of about 2 microns.

The slow release of macromolecules from implants has been demonstrated in experiments where macromolecules were dissolved in aqueous suspensions of crystallized dextran microparticles or their aggregates before injections. Figure 4 shows an implant containing fluorescently labeled macromolecules (FITC-dextran, MW 500 kDa) and slow release of the macromolecules from the implant into a mouse muscle tissue on the 14^{th} day after the intermuscular injection.

### B. Two-phase system

Self assembled structures based on crystallized dextran microparticles and their aggregates may be formed based on two phase systems.

For example, in the case of oil, a special kind of structure can be formed where the oil core is surrounded with a shell composed of crystallized dextran microparticles or aggregates thereof dispersed in water or aqueous solutions of organic polymers such as polysaccharides (e.g. dextrans). The structure described can be designated as a capsule. It should be noted that the shell may comprise a roughly spherical shaped shell which results when the capsule is surrounded by tissue. However, when the capsule is located near a barrier, such as a substrate, bone or intestine wall, the capsule may comprise a core located between one or more walls of microparticles on one side and the barrier on the other side. Furthermore, while oil is used as an illustrative example, the core may comprise other materials, such as other polymers, cells, etc.

To form the capsule structure, two-phase aqueous systems are applied. When aqueous solutions of different polymers are mixed above certain concentrations they frequently form immiscible-liquid two-phase solutions. Each of the phases usually consists of more than 90% water and can be buffered and made isotonic. If a cell or particle suspension is added to such a system, the cells or particles are frequently found to have partitioned unequally between phases. This preferential partition behavior can be used as a basis for separation procedures for differing cell populations or particles since partition in these systems is determined directly by cell or particle surface properties. Cells or particles which do not have identical surface properties exhibit sufficiently different partition behavior.

The competitive adsorption of the two polymer phase depends on the chemical nature of the polymers. A two-phase polymer method has been applied to separate or partition cells, proteins, nucleic acids and minerals ("Partitioning in Aqueous Two-Phase Systems", 1985, eds., H. Walter, D. Brooks, and D. Fisher, publs. Academic Press).

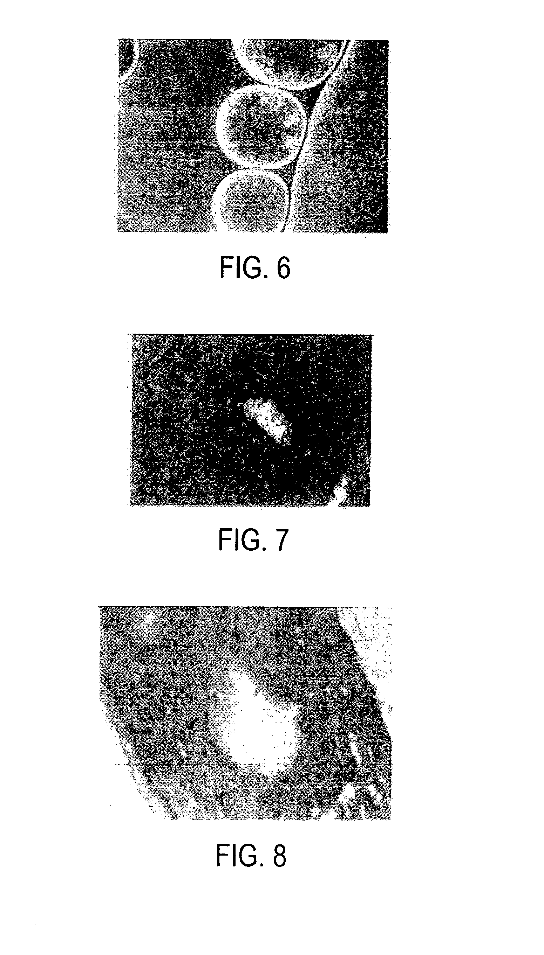

The experiments with the distribution of crystallized dextran microparticles in phase systems derived from, for instance, dextran/polyethylene glycol (PEG) mixtures, revealed that the dextran microparticles prefer to be in the dextran phase, while another PEG phase can be dispersed in this dextran phase to form a W/W emulsion and vice versa in the case when the volume of the PEG phase is bigger than the volume of the dextran phase, as shown in Figures 5 and 6.

Figure 5 is a photograph of an emulsion of aqueous solution of PEG in aqueous solution of dextran containing crystallized dextran microparticles. In the structure of Figure 5, the volume of the PEG phase is less than the volume of the dextran phase. The dextran phase contains the dextran and the crystallized dextran microparticles. Thus, the PEG phase forms into one or more sphere shaped cores surrounded by dextran / dextran microparticle shells (i.e., a closed pore structure).

Figure 6 is a photograph of an emulsion of aqueous solution of dextran containing crystallized dextran microparticles in aqueous solution of PEG, where the volume of the PEG phase is greater than the volume of the dextran phase. In this case, the dextran phase forms into one or more sphere shaped cores containing the dextran microparticles surrounded by a PEG phase (i.e., an open pore structure that is forming in vivo while PEG dissipates in tissue liquid). As can be seen in Figure 6, the smaller volume (droplet) dextran phase forms into a large spherical dextran / dextran microparticle core (bottom right of Figure 6) to which smaller spheres comprising dextran / dextran microparticles are joining and fuse with.

Thus, when the ratio of the volume of the first phase (such as the PEG phase and its inclusions, such as a therapeutic agent) to the volume of the second phase (such as the dextran phase and its inclusions, such as the dextran microparticles) is less than one, then the capsule forms by self assembly with a first phase core surrounded by a second phase shell. If the composition contains a therapeutic agent, such as insulin, which prefers to partition into the PEG phase, and the dextran microparticles which prefer to partition into the dextran phase, then the therapeutic agent selectively partitions into the PEG core while the microparticles selectively partition into and form the shell around the PEG core by self assembly.

The emulsion can be prepared by the mixing of separately prepared dextran and PEG phases and both can be suspensions of different types of particles that prefer to be in the PEG phase or in the dextran phase respectively. The principle is that the partition of particles into different polymer phases depends on their surface structure and interfacial energy of the particles in the polymer solutions.

Injection of aqueous two phase systems containing crystallized dextran microparticles into tissues of experimental animals revealed the formation of implants with the capsule structure as shown in Figures 7 and 8. The volume of the dextran phase is greater than the volume of the PEG phase in the two-phase system. Both Figures 7 and 8 show that a capsule with a PEG core and a dextran/dextran microparticle shell forms by self assembly in vivo (i.e., after injection into mammal tissue). The shell comprises macroporous regions between adjacent microparticles as well as microporous regions in the microparticles themselves.

A non limiting example of a method of forming a capsule structure from a two phase system is as follows. 10 g of dextran T40 (40 kDa molecular weight) and 2 g of PEG are dissolved in 88 ml of (Actrapid®) insulin solution containing 1,000 Ul to which 25 g of crystallized dextran microparticles are added. These steps are performed under laminar flow conditions. The mixture is stirred on a magnetic stirrer at 100 rpm at room temperature for 30 minutes to form a homogeneous mixture (i.e., a suspension). 1.0 g of the suspension contains 8 Ul of insulin.

It should be noted that the dextran microparticles may be prepared from a different molecular weight dextran solution than the dextran solution which is provided into the two phase system. Thus, the crystallized dextran microparticles may be formed in a lower molecular weight dextran solution, such as a 2 to 20 kDa solution, than the dextran solution which is provided in the two phase system, which may be a 40 to 500 kDa dextran solution, such as a 40 to 75 kDa solution. This is advantageous because the higher molecular weight dextran solutions, such as 40 and 70 kDa solutions, have received wider regulatory approval and can be used to form a shell of a capsule at lower concentrations. The lower molecular weight solutions may be used to decrease the crystallization time without the lower molecular weight dextran solution actually being provided in vivo. Furthermore, lower molecular weight microparticles may dissolve easier in vivo.

The capsule structure formed from a two phase system is advantageous because it allows for a more even and prolonged release of the therapeutic agent from the core than from a composition comprising a single phase containing the microparticles. Furthermore, it is believed that by using the capsule structure, a lower amount of microparticles may be needed to achieve the same or better timed release of a therapeutic agent than if a single phase system is used. Furthermore, by controlling the amount of microparticles in the two phase system, it is believed that the thickness of the microparticle shell may be controlled. A thicker shell results from a larger amount of microparticles in the two phase system. Thus, the amount, duration and/or timing of the release of the therapeutic agent from the capsule core may be controlled by controlling the thickness of the shell. Therefore, the release profile of the therapeutic agent may be customized for each patient or groups of patients.

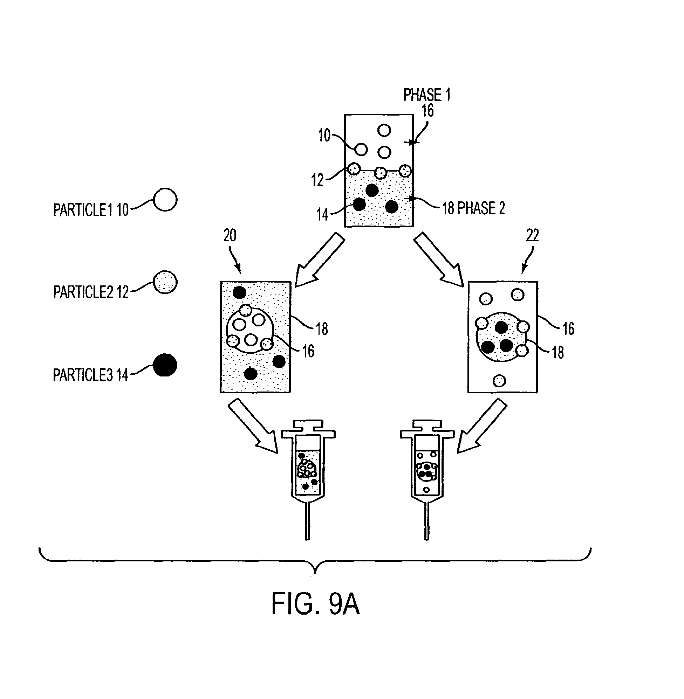

It should be noted that while PEG and dextran are used as examples of the materials of the two phases, any other suitable materials which show the following partition behavior may be used instead. Figure 9A schematically illustrates partition behavior of different types of particles in an aqueous two phase system. For example, three types of molecules or molecular aggregates, which are preferably particles 10, 12 and 14, and two phases 16 and 18 are shown in Figure 9A. However, there may be two, or more than three types of particles. The particles may be microparticles such as microspheres or nanospheres prepared from organic and/or inorganic materials, liposomes, living cells, viruses and macromolecules. The first type particles 10 preferentially segregate into the first phase 16. The second type particles 12 preferentially segregate to the boundary of the first 16 and second 18 phases. The third type particles 14 preferentially segregate into the second phase 18. Thus, by analogy to the previous non-limiting example, the first particles 10 may comprise a therapeutic agent, the second 12 and/or the third 14 particles may comprise crystallized dextran microparticles, the first phase 16 may comprise a PEG phase and the second phase 18 may comprise a dextran phase.

If a smaller amount of the first phase 16 is provided into a larger amount of the second phase 18, as shown in area 20 of Figure 9A, then a capsule type structure forms comprising discreet spheres of the first phase 16 containing a concentration of the first type particles 10, located in a second phase 18. The second type particles 12 may be located at the interface of the phases 16, 18 and act as a shell of the capsule. Particles 14 are dispersed in the second phase 18 and/or form a shell of the capsule.

In contrast, if a smaller amount of the second phase 18 is provided into a larger amount of the first phase 16, as shown in area 22 of Figure 9A, then a capsule type structure forms comprising discreet spheres of the second phase 18 containing a concentration of the third type particles 14, located in a first phase 16. The second type particles 12 may be located at the interface of the phases 16, 18 and act as a shell of the capsule. Particles 10 are dispersed in the first phase 16 and/or form a shell of the capsule. The two phase systems 20 and 22 may be used as an implant, such as by being delivered into a mammal, such as an animal or human. Thus, the capsule forms a structured, three dimensional implant, with the core acting as a reservoir or depot for controlled release of the therapeutic agent through the shell. In contrast, an implant with an even distribution of microparticles is an unstructured implant. It should be noted that the structure formed for orally delivered two phase systems may be generally described as a structured suspension comprising a dispersed PEG phase and a continuous dextran phase.

Furthermore, particles 10, 12 and 14 may be substituted by a liquid material (e.g. oils) or macromolecules which selectively partition into one of the phases. For example, a therapeutic agent, such as insulin, may be partitioned in PEG phase of the PEG/dextran two phase system. Since insulin selectively partitions into the PEG phase, the PEG phase forms an insulin containing core of a capsule structure. It should be noted that while certain particles and therapeutic agents selectively partition, the term "selectively partitioned" does not necessarily mean that 100 percent of the particles or therapeutic agent partition into one of the phases. However, a majority of the selectively partitioned specie, preferably 80% of the partitioned specie, partitions into one of the phases. For example, while a majority of insulin partitions into the PEG phase, a portion of insulin may remain in the dextran phase.

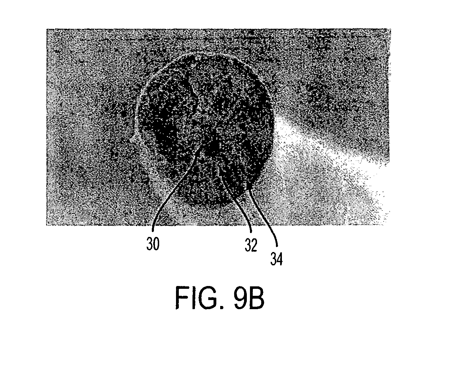

Figure 9B illustrates a scanning electron microscope image of a cross section of an implant structure based on the two phase system schematically illustrated in Figure 9A. A two phase aqueous composition comprising a first dextran phase, a second PEG phase and crystallized dextran microparticles was injected into sepharose gel. This gel's composition mimics mammal tissue by stopping crystallized dextran microparticles diffusion from the injection side. The image in Figure 9B illustrates the formation of a core-shell implant structure. The core comprises regions 30 and 32 surrounded by a shell 34. Region 30 is a void that is filled with a PEG phase region prior to cutting the gel for cross sectional SEM imaging. The PEG phase region drips out of the gel when the gel is cut during cross sectioning. Region 32 is an outer portion of the core comprising PEG droplets located in the crystallized dextran microparticles. Region 34 is the shell comprising the crystallized dextran microparticles which surrounds and holds in place the PEG containing core.

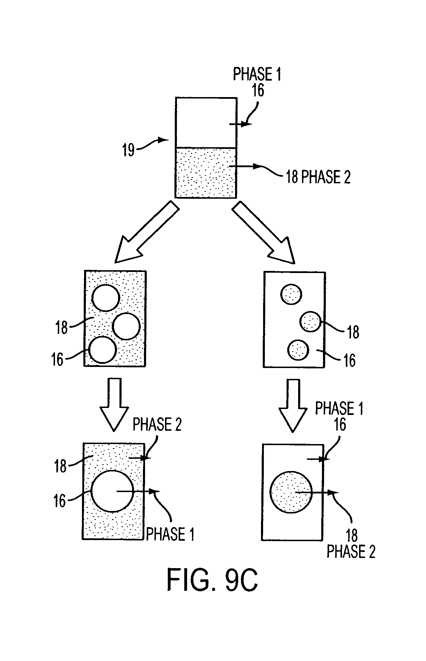

Without wishing to be bound by a particular theory, the present inventor believes that the core-shell structure shown in Figure 9B forms by self assembly as shown schematically in Figure 9C. While the first 16 and second 18 phases, such as aqueous solutions of different, incompatible polymers, are in a suitable storage container 19, such as in a glass beaker or vial, one phase 16 rises above the other phase 18. When the two phase composition is injected into a material which restricts free flow of the phases 16 and 18, such as mammal tissue or a substrate material, such as a gel which mimics the tissue, the composition self assembles into the core-shell structure. First, the phase that is present in the smaller volume forms into approximate spherical shapes, as shown in the middle portion of Figure 9C. Then the spherical shapes join to form approximately spherical cores of one phase surrounded by shells of the other phase, as shown in the bottom of Figure 9C. While a two phase system example of a multiphase system has been illustrated, the multiphase system may have more than two phases if desired.

### C. Oral insulin delivery vehicle

The present inventor discovered that a composition of porous (i.e., microporous) microparticles may be used as a vehicle for oral delivery of insulin. The porous microparticles may be any suitable porous microparticles which enable oral administration of insulin with a significant reduction in blood glucose, such as at least a 5% reduction, for example at least a 30% reduction within 60 minutes of oral administration. Preferably, the microparticles are bioadhesive particles, such as particles which adhere at least temporarily to mammal intestine walls, to allow insulin deliver through the intestine wall. Most preferably, the porous microparticles comprise the crystallized dextran microparticles described above.

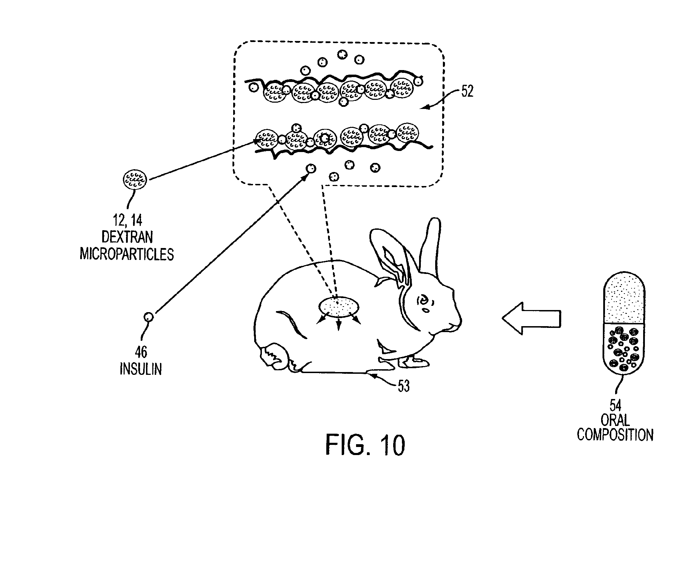

In one preferred embodiment of the present invention shown in Figure 10, the present inventor has discovered that an aqueous suspension of crystallized dextran microparticles 12, 14 and insulin 46 orally administered to mammals 53, such as rabbits, was about equally as effective in reducing blood glucose levels as an intramuscular injection of insulin alone. Figure 10 schematically illustrates the insulin 46 permeating through mammal 53 intestine 52 walls from the orally administered composition 54 comprising the microparticles. Since rabbits are a common model for humans in drug testing, the present inventor believes that a liquid or solid composition 54 comprising crystallized dextran microparticles and insulin, such as an aqueous suspension, a solution, a tablet or a capsule, would also be effective in reducing blood glucose levels in human beings when orally administered.

The following examples illustrate oral insulin delivery using crystallized dextran microparticles. The study involved Chinchilla rabbits (2.3±0.2 kg) and the observation of their response to orally administered aqueous suspensions consisting of crystallized dextran microparticles prepared according to the method described herein and human recombinant insulin.

3.0 g of Dextran T20 (Pharmacia, Uppsala, Sweden) was dissolved in 2.0 g of water and placed in box at temperature 60° C. Three hours later, crystallized dextran microparticles were washed by centrifugation at 3,000 g with 3 × 5.0 ml of water. Then the crystallized dextran microparticles were suspended in 2.0 ml of water and allowed to dry at room temperature. The resulting dry powder was used to prepare an insulin containing suspension for the oral insulin delivery experiment. Insulin containing suspensions were prepared by the mixing of 250 mg of the microparticles; 0.3 ml (12 Ul) or 0.6 ml (24 Ul) of insulin (NovoNordisk Actrapid HM Penfill, 40 Ul/ml); and distilled water to reach a volume of 2.0 ml.

Samples of the suspension (2.0 ml) were introduced into the rabbits' throats with a catheter followed by the introduction of 10.0 ml of drinking water. Animals were not fed for 3 hours before the suspension's introduction. Samples of blood were taken from the rabbit's ear vein and analyzed for glucose concentrations. Blood glucose was measured using the glucose oxidase method on a "One-touch System Glucose Analyzer" (Lifescan Johnson & Johnson, Milpitas, CA, USA) after proper calibration.

Examples 1 to 8 are comparative examples involving eight rabbits. Examples 9 to 14 are examples according to the present invention involving five rabbits.

In comparative examples 1 and 2 (control experiment #1 summarized in Table I) an aqueous solution of human recombinant insulin was introduced intra muscularly into two rabbits at a dose of 12 Ul per animal. In comparative examples 3 and 4 (control experiment #2 summarized in Table II), the rabbits remained intact (i.e., no insulin or other injection was provided to the two rabbits). In comparative examples 5 and 6 (control experiment #3 summarized in Table III), a suspension of the crystallized dextran microparticles without insulin was provided orally to two rabbits. In comparative examples 7 and 8 (control experiment #4 summarized in Table IV), a suspension of the commercially obtained Sephadex G-150 microparticles with insulin (24 Ul) was provided orally to two rabbits. According to the Amersham Biosciences website, Sephadex® G-150 microparticles are beaded gel microparticles having a diameter of 20 to 150 microns, prepared by cross linking dextran with epichlorohydrin. In examples 9-14 according to a preferred embodiment of the present invention (summarized in Table V), a suspension of crystallized dextran microparticles with insulin (24 Ul) was provided orally to five rabbits. The results are summarized in Tables I-V below.

**Table I**

| Rabbit/ Example # | Insulin dose | 0 min mg/dl | 30 min mg/dl | 60 min mg/dl | 90 min mg/dl | 120 min mg/dl |
|---|---|---|---|---|---|---|
| #1 | 12 Ul i.m. | 91 | 68 | 58 | 49 | 51 |
| #2 | 12 Ul i.m. | 87 | 65 | 64 | 57 | 58 |

**Table II**

| Rabbit/ Example # | Insulin dose | 0 min mg/dl | 30 min mg/dl | 60 min mg/dl | 90 min mg/dl | 120 min mg/dl |
|---|---|---|---|---|---|---|
| | | | | | | |
| #3 | 0.0 | 98 | 87 | 87 | 89 | 86 |
| #4 | 0.0 | 88 | 90 | 91 | 94 | 87 |

**Table III**

| Rabbit/ Example # | Insulin dose | O min mg/dl | 30 min mg/dl | 60 min mg/dl | 90 min mg/dl | 120 min mg/dl |
|---|---|---|---|---|---|---|
| #5 | 0.0 | 92 | 99 | 94 | 95 | 92 |
| #6 | 0.0 | 90 | 93 | 93 | 93 | 92 |

**Table IV**

| Rabbit/ Example # | Insulin dose | O min mg/dl | 30 min mg/dl | 60 min mg/dl | 90 min mg/dl | 120 min mg/dl |
|---|---|---|---|---|---|---|
| #7 | 24 Ul per os | 85 | 82 | 86 | 81 | 83 |
| #8 | 24 Ul per os | 84 | 75 | 86 | 76 | 77 |

**Table V**

| Rabbit/ Experiment # | Insulin dose | O min mg/dl | 30 min mg/dl | 60 min mg/dl | 90 min mg/dl | 120 min mg/dl |
|---|---|---|---|---|---|---|
| #9 | 24 Ul per os | 94 | 68 | 59 | 58 | 57 |
| #10 | 24 UI per os | 93 | 64 | 52 | 54 | 52 |
| #11 | 24 Ul per os | 78 | 52 | 51 | 49 | 48 |
| #12 | 24 Ul | 92 | 64 | 52 | 53 | 47 |
| | per os | | | | | |
| #13 | 24 Ul per os | 89 | 53 | 48 | 38 | 49 |
| #14 | 24 Ul per os | 97 | 60 | 38 | 54 | 52 |

The data in Tables I-V show that average reduction of sugar (i.e., glucose) in the blood of mammals is comparable when 12 Ul of insulin is administered by intramuscular injection (examples 1-2) and 24 Ul of insulin is administered per os (i.e., orally) with crystallized dextran microparticles (examples 9-14). The maximum glucose reduction was about 35 to about 60 percent at 60 min after oral administration. The concentration profile of glucose is practically the same in both the injection and oral delivery modes. It should be noted that other amounts of insulin may be administered. For example, 30 Ul of insulin may be administered. In general, oral administration of two to three times the insulin compared to the amount of injected insulin produces a similar drop in blood sugar for up to three hours.

It is a well known fact that insulin by itself is degraded by intestinal enzymes and is not absorbed intact across the gastrointestinal mucosa (Amidon GL, Lee HJ, Absorption of peptide and peptidomimetic drugs, Ann. Rev. Pharmacol. Toxicol. 1994; 34: 321-41). However, examples 9-14 show that crystallized dextran microparticles can be used as a vehicle for oral delivery of proteins, such as insulin because the hypoglycemia effect received was significant. Without wishing to be bound by a particular theory or mode of action, the present inventor believes that the use of the porous, crystallized dextran microparticles as an insulin delivery matrix in an aqueous suspension protected the insulin from significant degradation by intestinal enzymes and allowed the insulin to be absorbed intact across the gastrointestinal mucosa. The insulin may be located in micropores in the microporous microparticles and/or in macropores between the microparticles. In contrast, the use of cross-linked Sephadex G-150 dextran microparticles with insulin (Table IV, examples 7-8) did not produce an appreciable reduction in blood glucose concentration.

As provided in examples 9-14, the blood glucose concentration in the mammal is lowered by at least 5 percent, preferably at least 30 percent, 60 minutes after administering the composition containing the crystallized dextran microparticles and insulin to the mammal (i.e., the blood glucose value in the mammal at 60 minutes after administration of the suspension is at least 5 percent, preferably at least 30 percent lower than that measured right before administration of the suspension). Preferably, the blood glucose concentration in the mammal is lowered by at least 5 percent, such as at least 30 percent, preferably by about 35 to about 40 percent 30 minutes after administering the composition to the mammal. Preferably, the blood glucose concentration in the mammal is lowered by about 35 to about 60 percent, for example 35 to 45 percent 60 minutes after administering the suspension to the mammal. More preferably, the blood glucose concentration in the mammal is lowered during the entire period ranging from 30 to 240 minutes, such as 30 to 120 minutes, after administering the composition to the mammal compared to the blood glucose concentration right before administration. For example, the blood glucose concentration in the mammal is preferably lowered by at least 10 percent, preferably at least 30 percent, more preferably by at least 35 percent, such as by 35 to 45 percent during a period ranging from 30 to 240 minutes, preferably 30 to 120 minutes after administering the composition to the mammal.

Thus, a preferred embodiment of the present invention provides a method of lowering blood glucose in a mammal by orally administering a therapeutically effective amount of a composition comprised of crystallized dextran microparticles and insulin. A "therapeutically effective" amount of the compositions can be determined by prevention or amelioration of adverse conditions or symptoms of diseases, injuries or disorders being treated. Preferably, the composition comprises an aqueous suspension of crystallized dextran microparticles having an average diameter of about 0.5 to about 5 microns and insulin. Furthermore, the microparticles are preferably porous microparticles which are crystallized prior to adding the insulin to the suspension, such that the insulin is located in contact with a surface of the microparticles and/or in pores of the microparticles.

The crystallized microparticles preferably are comprised of dextran molecules (i.e., polymer molecules) that are held together by a plurality of hydrogen bonds, Van Der Waals forces and/or ionic bonds and having substantially no covalent bonds between the dextran molecules. Thus, the molecules in the microparticles are preferably not intentionally cross-linked (i.e., a cross linking step is not carried out) and the microparticles contain no covalent bonds between molecules or less than 10% covalent bonds between molecules.

While a one phase composition 54 comprising insulin and microparticles is illustrated in Figure 10, a two phase composition, described above and illustrated in Figures 7, 8, 9A, 9B and 9C may also be used. For example, a two phase composition comprising a dextran phase, a PEG phase, crystallized dextran microparticles and insulin may be used. In vivo, the composition has a self assembled capsule structure comprising a crystallized dextran microparticle containing wall or shell and a PEG and insulin containing core.

Preferably, the mammal receiving the oral administration of insulin comprises a human in need of lowering blood glucose, such as a human suffering from diabetes. Thus, the preferred embodiment of the present invention provides a method of treating diabetes in a human in need of the treatment by orally administering the suspension of insulin and crystallized dextran microparticles described above.

Any therapeutically effective amount of insulin may be administered to the mammal. The amount of insulin may vary based on the type of mammal (i.e., human or rabbit), the weight of the mammal, the composition of the suspension, the amount of desired reduction of blood glucose and other factors. One non-limiting example of insulin content in the suspension is about 10 Ul to about 2,500 Ul of human recombinant insulin per one gram of suspension, such as about 12 Ul to about 30 Ul, such as 24 Ul of human recombinant insulin. However, this amount may vary as desired.

The present invention should not be considered limited to the preferred embodiments described above. Other matrix material may be used for oral insulin delivery, such as organic or inorganic microporous particles. Preferably, the particles are microparticles which enhance insulin penetration through gastrointestinal mucosa and/or which stabilize the composition. Furthermore, while the suspension preferably contains only water solvent; a matrix; and an insulin solution or suspension; the delivery system may also contain additional materials. For example, the composition may contain a second phase such as the PEG phase of a two phase system. Thus, another preferred aspect of the present invention includes a method of lowering blood glucose in a mammal comprising of orally administering a composition comprising a therapeutically effective amount of insulin and a matrix material to the mammal to lower blood glucose of the mammal by at least 30 percent 60 minutes after administering the suspension to the mammal. In another preferred aspect of the present invention, a method of administering a suspension to a mammal comprised of orally administering an aqueous suspension of crystallized dextran microparticles and a therapeutically effective amount of insulin to the mammal.

As noted above, the crystallized dextran microparticles used as a matrix material for oral administration of insulin or other protein based drugs may be made by any suitable method (see Figure 1, for example). Preferably, the microparticles are made by the process of any of the preferred embodiments described herein. Preferably, but not necessarily, the microparticles are formed in an aqueous solution without using an organic solvent. Thus, in a preferred aspect of the present invention, a therapeutically effective amount of insulin and the crystallized dextran microparticles are combined in water after the microparticles have been crystallized to form an aqueous suspension of insulin and crystallized dextran microparticles. The microparticles may be added to the water before, at the same time and/or after adding the insulin to the water. Furthermore, the microparticles may be orally administered to a mammal in the solvent in which they were formed. Alternatively, they may be removed from the solvent in which they were formed and placed into water or other aqueous solutions for oral administration or dried and provided in solid form, such as tablet or capsule, for oral administration.

The aqueous suspension of crystallized dextran microparticles and a therapeutically effective amount of insulin (or other suitable suspensions of insulin and a matrix material, such as a suspension of insulin and microporous microparticles) is preferably provided as a dosed pharmaceutical composition which is dosed for oral administration to a human. In one preferred aspect of the present invention, the composition is located in a vessel in an amount dosed for a single oral administration to a human. The vessel may comprise any container which may hold a suspension, such as a plastic or glass bottle, a tube, a dropper, a spray nozzle, a pouch and/or other suitable vessels. This vessel contains an amount of suspension sufficient for a single oral dose of the composition.

In another preferred aspect of the present invention, the composition is located in any suitable vessel in an amount suitable for multiple oral administration doses. The vessel contains an instruction for oral dosage administration to a human. The instruction may be printed on the vessel, such being as printed directly on the vessel or on an label attached to the vessel, or enclosed with the vessel, such being printed on a sheet of paper enclosed with the vessel in a cardboard box or in a pharmacy envelope. The instructions may describe the amount of the composition that should be taken with each dose, the frequency that the dose should be taken, how to measure the dose of the composition for oral administration and/or any other suitable oral drug instructions for an administering health care practitioner and/or a patient in need of the drug. Alternatively, the instructions may comprise directions for electronically or audibly accessing the dosing and administration instructions, such as a link to a website containing the instructions or a telephone number or recording where the instructions are provided audibly.

In another preferred aspect of the present invention, the aqueous suspension of crystallized dextran microparticles and a therapeutically effective amount of insulin located in a vessel are provided in a pharmaceutical composition kit with instructions for oral administration of the composition to a human in need thereof. The kit may comprise instructions printed on the vessel or on a label attached to the vessel or a sheet of paper enclosed with the vessel, such as in a cardboard box or pharmacy envelope including a bottle (i.e., vessel) and the sheet of instructions.

It should be noted that the composition for oral administration may be in the form of an aqueous suspension, but other delivery forms may be used to lower the blood glucose in a mammal. For example, the porous crystallized dextran microparticles and the insulin may be orally administered in the form of a tablet or a capsule.

To orally administer the composition in solid form to a mammal, such as a human, the solution of crystallized dextran microparticles and insulin is first dried, such as freeze dried, to form a powder. The powder may then be compressed into a tablet, along with optional pharmaceutically acceptable excipients or placed into a pharmaceutically acceptable capsule.

### D. Materials

In the preferred embodiments of the present invention, the therapeutic agent comprises insulin. In other words, the therapeutic agent may consist essentially of insulin alone or comprise insulin in combination with another agent. The term "insulin" shall be interpreted to encompass insulin analogs, natural extracted human insulin, recombinant produced human insulin, insulin extracted from bovine and/or porcine sources, recombinant produced porcine and bovine insulin and mixtures of any of these insulin products. The term is intended to encompass the polypeptide normally used in the treatment of diabetics in a substantially purified form but encompasses the use of the term in its commercially available pharmaceutical form, which includes additional excipients. The insulin is preferably recombinant produced and may be dehydrated (completely dried) or in solution.

The terms "insulin analog," "monomeric insulin" and the like are used interchangeably herein and are intended to encompass any form of "insulin" as defined above, wherein one or more of the amino acids within the polypeptide chain has been replaced with an alternative amino acid and/or wherein one or more of the amino acids has been deleted or wherein one or more additional amino acids has been added to the polypeptide chain or amino acid sequences, which act as insulin in decreasing blood glucose levels. In general, the term "insulin analogs" of the preferred embodiments of the present invention include "insulin lispro analogs," as disclosed in U.S. Pat. No. 5,547,929, incorporated hereinto by reference in its entirety; insulin analogs including LysPro insulin and humalog insulin, and other "super insulin analogs", wherein the ability of the insulin analog to affect serum glucose levels is substantially enhanced as compared with conventional insulin as well as hepatoselective insulin analogs which are more active in the liver than in adipose tissue. Preferred analogs are monomeric insulin analogs, which are insulin-like compounds used for the same general purpose as insulin, such as insulin lispro, i.e., compounds which are administered to reduce blood glucose levels.

The term "analog" refers to a molecule, which shares a common functional activity with the molecule to which it is deemed to be comparable and typically shares common structural features as well.

The term "recombinant" refers to any type of cloned therapeutic expressed in prokaryotic cells or a genetically engineered molecule, or combinatorial library of molecules which may be further processed into another state to form a second combinatorial library, especially molecules that contain protecting groups which enhance the physicochemical, pharmacological, and clinical safety of the therapeutic agent.

The term dextran microparticles includes unsubstituted dextran microparticles and substituted dextran microparticles. For example, substituted dextran microparticles include dextran substituted with a suitable group, such as a methyl group, up to a degree which does not hamper crystallization of the dextran microparticles, such as up to 3.5 or less percent branching. The average microparticle diameter is preferably about 0.5 to about 5 microns, more preferably about 1 to about 2 microns.

Furthermore, while porous non cross-linked dextran microparticles, such as crystallized microparticles, are preferably used with the therapeutic agent, other suitable organic or inorganic microparticles may be used instead, such as other polymer microparticles including polysaccharides, PLA, PLGA, PMMA, polyimides, polyesters, acrylates, acrylamides, vinyl acetate or other polymeric materials, biomaterial particles such as alginate and cells, or inorganic particles, such as silica, glass or calcium phosphates. Preferably the microparticles are biodegradable. Preferably, porous microparticles are used. Most preferably, the microparticles have sufficient porosity to contain the therapeutic agent within the pores and to provide a timed release of the therapeutic agent from the pores. In other words, the therapeutic agent is released over time from the pores, such as in over 5 minutes, preferably in over 30 minutes, most preferably in over one hour, such as in several hours to several days, rather than all at once. Thus, the particle material, pore size and pore volume can be selected based on the type of therapeutic agent used, the volume of therapeutic agent needed for delivery, the duration of the delivery of the therapeutic agent, the environment where the therapeutic agent will be delivered and other factors.

Thus, in a preferred aspect of the present invention, the therapeutic agent is located at least partially in the pores of the porous microparticles. Preferably, the therapeutic agent is not encapsulated in the microparticle (i.e., the microparticle does not act as a shell with a therapeutic agent core inside the shell) and is not attached to the surface of the microparticle. However, if desired, a portion of the therapeutic agent may also be encapsulated in a microparticle shell and/or is attached to the surface of the microparticle in addition to being located in the pores of the microparticle. The location of the therapeutic agent in the pores provides an optimum timed release of the therapeutic agent. In contrast, the therapeutic agent attached to the surface of the microparticle is often released too quickly, while the therapeutic agent encapsulated in the microparticle is often not released soon enough and is then released all at once as the microparticle shell disintegrates. In a two phase system, at least 80% of the therapeutic agent is preferably located in a core surrounded by a wall or shell comprising the microparticles.

### E. Methods of Making

The microparticles may be formed by any suitable method. Preferably, the microparticles are combined with the therapeutic agent after the microparticles are formed. Thus, the microparticles, such as the crystallized dextran microparticles are formed by any suitable method and then the therapeutic agent and the microparticles are combined by any suitable method. In contrast, in some prior art methods, the therapeutic agent is encapsulated into a microparticle shell by providing the particle precursor material and the therapeutic agent into a solution and then crystallizing or cross-linking the precursor material, such as a monomer or oligomer material, to encapsulate a therapeutic agent core into a microparticle shell.

Preferably, the therapeutic agent is provided into the pores of the porous microparticles after the microparticles are formed. Thus, the porous microparticles are first formed and then the therapeutic agent is provided into a solution containing the microparticles to allow the therapeutic agent to permeate into the pores of the microparticles. Of course, some of the therapeutic agent may also become attached to the surface of the microparticle in this process.

Thus, a method to manufacture non cross-linked, porous crystallized dextran microparticles includes preparation of a dextran solution, such as an aqueous dextran solution, conducting a crystallization process to form crystallized porous dextran microparticles, and if desired, isolating crystallized porous dextran microparticles from the solution. A therapeutic agent is then permeated into the pores of the microparticles by providing the therapeutic agent into the crystallization solution containing the microparticles or by providing the isolated microparticles and the therapeutic agent into a second solution, such as a second aqueous solution. For example, crystallized dextran microparticles may be formed in a first, low molecular weight dextran aqueous solution, such as a 2 to 20 kDa dextran solution. The microparticles are then removed from the first solution and then placed into a second dextran aqueous solution having a higher molecular weight dextran, such as a 40 to 500 kDa solution, for example, a 40 to 75 kDa solution. The second solution may comprise a first phase of a two phase system, which is then combined with a second phase, such as a PEG phase containing a therapeutic agent. A similar method may be used with other porous microparticles, where a therapeutic agent is then permeated into the pores of the microparticles after the porous microparticles are formed by any suitable microparticle formation method, including, but not limited to crystallization. The components of the composition such as insulin, microparticles and one or more aqueous phases may be combined in any suitable order sequentially or simultaneously.

Preferably, the microparticles are formed by self assembly from a solution that does not contain organic solvents and organic reaction promoters which leave an organic residue in the microparticles. Thus, for example, the dextran microparticles are preferably formed by self assembly from an aqueous dextran solution. However, if desired, organic solvents and/or organic reaction promoters may also be used. In this case, the microparticles may be purified prior to subsequent use to remove the harmful organic residue.

As described above, the capsule structure having a first phase core and a second phase wall or shell may be formed in vivo or in vitro from a two phase composition. The composition may be a dried powder, such as freeze dried and stored as a powder or porous cake. When the composition is ready to be administered to a mammal, it is hydrated and administered to a mammal orally.

Preferably, the composition which includes the microparticles and the therapeutic agent is a flowable colloidal system. Examples of flowable colloidal systems include emulsions and suspensions. In contrast, some prior art compositions include a therapeutic agent in a dextran hydrogel or in a cross-linked dextran matrix. A dextran hydrogel and a cross-linked dextran matrix are not flowable compositions if not specifically prepared.

In another preferred aspect of the present invention, the microparticles comprise microparticles which are adhesive to mammalian mucosa. Preferably the adhesive microparticles are porous microparticles described above. This further improves the effective delivery of the therapeutic agent.

In another preferred aspect of the present invention, the microparticles comprise microparticles whose surface has been specially modified to enhance the adhesion of the therapeutic agent to the microparticle surface and to optimize the delivery of the therapeutic agent. The microparticle surface may contain any suitable modification that would increase the adhesion of the therapeutic agent.

This invention also provides for the use of a composition comprising crystallized dextran microparticles and insulin in the manufacture of an oral medicament for lowering blood glucose in a mammal.

This invention also provides for the use of a composition comprising a therapeutically effective amount of insulin and a matrix material in the manufacture of an oral medicament for lowering blood glucose in a mammal.

This invention also provides for the use of an aqueous suspension of crystallized dextran microparticles and insulin in the manufacture of an oral medicament for administering a suspension to a mammal.

The foregoing description of the invention has been presented for purposes of illustration and description. It is not intended to be exhaustive or to limit the invention to the precise form disclosed, and modifications and variations are possible in light of the above teachings or may be acquired from practice of the invention. The drawings and description were chosen in order to explain the principles of the invention and its practical application. It is intended that the scope of the invention be defined by the claims appended hereto, and their equivalents.

All of the publications and patent applications and patents cited in this specification are herein incorporated in their entirety by reference.

## Claims

1. A method of lowering blood glucose in a mammal, comprising orally administering a therapeutically effective amount of a composition comprising crystallized dextran microparticles and insulin to the mammal to lower blood glucose of the mammal.

2. The method of claim 1, wherein the composition comprises an aqueous suspension of crystallized dextran microparticles having an average diameter of about 0.5 to about 5 microns and the insulin.

3. The method of claim 2, wherein the method comprises orally administering the suspension to a human in need of lowering blood glucose.

4. A method of treating diabetes in a human in need thereof by orally administering the suspension of claim 2.

5. The method of claim 2, wherein the microparticles are porous microparticles which are crystallized prior to adding the insulin to the suspension, such that the insulin is located in contact with a surface of the microparticles or in pores of the microparticles.

6. The method of claim 2, wherein the therapeutically effective amount of insulin ranges from 10 to 2,500 Ul of human recombinant insulin per 1 gram of the suspension.

7. The method of claim 2, wherein the blood glucose concentration in the mammal is lowered by at least 5 percent 60 minutes after administering the suspension to the mammal.

8. The method of claim 7, wherein the blood glucose concentration in the mammal is lowered by about 30 percent to about 60 percent 60 minutes after administering the suspension to the mammal.

9. The method of claim 2, wherein the blood glucose concentration in the mammal is lowered by at least 5 percent 30 minutes after administering the suspension to the mammal.

10. The method of claim 2, wherein the blood glucose concentration in the mammal is lowered by at least 10 percent during a period ranging from 30 minutes to 240 minutes after administering the suspension to the mammal.

11. The method of claim 2, wherein the blood glucose concentration in the mammal is lowered during a period ranging from 30 minutes to 120 minutes after administering the suspension to the mammal.

12. The method of claim 1, wherein:
the composition comprises a two phase composition comprising a dextran phase and a PEG phase;
the insulin is selectively partitioned in the PEG phase and the microparticles are selectively partitioned in the dextran phase; and
the composition comprises a structure comprising a dispersed PEG phase and a continuous dextran phase when the composition is present in the mammal body.

13. A method of lowering blood glucose in a mammal, comprising orally administering a composition comprising a therapeutically effective amount of insulin and a matrix material to the mammal to lower blood glucose of the mammal by at least 5 percent 60 minutes after administering the suspension to the mammal.

14. The method of claim 13, wherein the composition comprises an aqueous suspension and the matrix material comprises crystallized dextran microparticles having an average diameter of about 0.5 to about 5 microns.

15. The method of claim 14, wherein the microparticles are porous microparticles which are crystallized prior to adding the insulin to the suspension, such that the insulin is located in contact with a surface of the microparticles or in pores of the microparticles.

16. The method of claim 13, wherein the method comprises orally administering the composition to a human in need of lowering blood glucose.

17. A method of treating diabetes in a human in need thereof by orally administering the composition of claim 16.

18. The method of claim 13, wherein the therapeutically effective amount of insulin ranges from 10 to 2,500 Ul of human recombinant insulin per 1 gram of suspension.

19. The method of claim 13, wherein the blood glucose concentration in the mammal is lowered by about 30 percent to about 60 percent 60 minutes after administering the composition to the mammal.

20. The method of claim 13, wherein the blood glucose concentration in the mammal is lowered by at least 5 percent 30 minutes after administering the composition to the mammal.

21. The method of claim 13, wherein the blood glucose concentration in the mammal is lowered by at least 10 percent during a period ranging from 30 minutes to 240 minutes after administering the composition to the mammal.

22. The method of claim 13, wherein the blood glucose concentration in the mammal is lowered during a period ranging from 30 minutes to 120 minutes after administering the composition to the mammal.

23. The method of claim 13, wherein the composition is in a form of a tablet or a capsule.

24. The method of claim 13, wherein:
the composition comprises a two phase composition comprising a dextran phase and a PEG phase;
the insulin is selectively partitioned in the PEG phase and the microparticles are selectively partitioned in the dextran phase; and
the composition comprises a structure comprising a dispersed PEG phase and a continuous dextran phase when the composition is present in the mammal body.

25. A method of administering a suspension to a mammal, comprising orally administering an aqueous suspension of crystallized dextran microparticles and a therapeutically effective amount of insulin to the mammal.

26. A dosed pharmaceutical composition, comprising crystallized dextran microparticles and a therapeutically effective amount of insulin, wherein the composition is dosed for oral administration to a human.

27. The composition of claim 26, wherein:
the crystallized dextran microparticles comprise dextran molecules held together by hydrogen bonds, Van Der Waals forces or ionic bonds and having substantially no covalent bonds between dextran molecules; and
the crystallized dextran microparticles are porous microparticles having an average diameter of about 0.5 to about 5 microns and, such that the insulin is located in contact with a surface of the microparticles or in pores of the microparticles.

28. The composition of claim 26, wherein the composition comprises an aqueous suspension of crystallized dextran microparticles and a therapeutically effective amount of insulin.

29. The composition of claim 26, wherein the composition is located in a vessel in an amount dosed for a single oral administration to a human.

30. The composition of claim 26, wherein the composition is located in a vessel with instruction printed on the vessel or enclosed with the vessel for oral dosage administration to a human.

31. The composition of claim 26, wherein the composition comprises a tablet comprising a pharmaceutically acceptable carrier medium, the crystallized dextran microparticles and the therapeutically effective amount of insulin.

32. The composition of claim 26, wherein the composition comprises a capsule comprising a pharmaceutically acceptable shell, the crystallized dextran microparticles and the therapeutically effective amount of insulin.

33. The composition of claim 26, wherein:
the composition comprises a two phase composition comprising a dextran phase and a PEG phase;
the insulin is selectively partitioned in the PEG phase and the microparticles are selectively partitioned in the dextran phase; and
the composition is adapted to form a structured suspension comprising a dispersed PEG phase and a continuous dextran phase.

34. A pharmaceutical composition kit, comprising:
an aqueous suspension of crystallized dextran microparticles and a therapeutically effective amount of insulin located in a vessel; and
instructions for oral administration of the composition to a human in need thereof.

35. A pharmaceutical kit, comprising:
a first means for orally administering to a mammal to lower blood glucose of the mammal by at least 30 percent 60 minutes after administering the suspension to the mammal; and
a storage vessel containing the first means.

36. A tablet comprising a pharmaceutically acceptable carrier medium, crystallized dextran microparticles and a therapeutically effective amount of insulin.

37. A capsule comprising a pharmaceutically acceptable shell, crystallized dextran microparticles and a therapeutically effective amount of insulin.

38. A method of making a dosed pharmaceutical composition, comprising:
providing crystallized dextran microparticles;
combining a therapeutically effective amount of insulin and the crystallized dextran microparticles in a solution after the microparticles have been crystallized to form a composition of insulin and crystallized dextran microparticles; and
dosing the composition for oral administration to a mammal.

39. The method of claim 38, wherein:
the composition comprises a flowable colloidal composition; and
the microparticles comprise crystallized dextran microparticles having an average diameter of 0.5 to 5 microns.

40. The method of claim 39, wherein:
the composition comprises a two phase composition comprising a dextran phase and a PEG phase;
the insulin is selectively partitioned in the PEG phase and the microparticles are selectively partitioned in the dextran phase; and
the composition comprises a structure comprising a dispersed PEG phase and a continuous dextran phase when the composition is present in the mammal body.
